# EUROPEAN PATENT APPLICATION

(11) **EP 3 705 184 A1**
(43) Date of publication of application: **09.09.2020**
(21) Application number: 18873731.6
(22) Date of filing: 30.10.2018
(51) Int. Cl.: B03C 1/28, B03C 1/00, B03C 1/01, C12Q 1/68, G01N 33/543

(54) **MAGNETIC PARTICLE COLLECTION METHOD AND TEST SET**

(30) Priority: 31.10.2017 JP 2017211095
(71) Applicant: Daiken Medical Co., Ltd., Izumi-shi Osaka 594-1157 (JP)
(72) Inventor: TOEI, Masashi, Yokohama-shi Kanagawa 222-0033 (JP); CHAYA, Yurie, Yokohama-shi Kanagawa 222-0033 (JP)
(74) Representative: dompatent von Kreisler Selting Werner - Partnerschaft von Patent- und Rechtsanwälten mbB
(86) International application number: PCT/JP2018/040348
(87) International publication number: WO 2019/088106

(57) **Abstract**

An object is to provide a means of improving the ease and rapidity of testing using magnetic particles. Included is an action step of causing a magnetic collecting means with recovered magnetic particles adhering thereto to contact a liquid that acts on a target component, and causing the liquid to act on the target component in a state in which the magnetic particles are adhering to the magnetic collecting means.

## Description

### [Technical Field]

The present invention relates to a testing tool using magnetic particles.

### [Background Art]

Magnetic particles are widely used as a means of separating a target component from a certain specimen. Specifically, technologies such as a method of using magnetic particles to separate a target component such as a gene or a biomarker from a liquid sample and detect the separated target component (for example, Patent Literatures 1 and 2), a method of separating a target component such as bacteria and detecting the separated target component (for example, Patent Literature 3), and a method of using magnetic particles to select desired cells and cultivate the selected cells (for example, Patent Literature 4) are widely known.

In these methods of the related art, as a means of recovering the magnetic particles capturing the target component, it is typical to use an external magnetic field such as a magnetic stand (for example, Patent Literature 5), or a magnetic field generating means such as an electromagnet (for example, Patent Literature 6).

### [Citation List]

### [Patent Literature]

[Patent Literature 1] Japanese Unexamined Patent Application Publication No. 2009-284834
[Patent Literature 2] Japanese Unexamined Patent Application Publication No. 2010-151678
[Patent Literature 3] Japanese Unexamined Patent Application Publication No. 2016-144445
[Patent Literature 4] Japanese Unexamined Patent Application Publication No. 2017-158585
[Patent Literature 5] Japanese Unexamined Patent Application Publication No. 2010-81915
[Patent Literature 6] Japanese Unexamined Patent Application Publication No. 2017-075938

### [Summary of Invention]

### [Technical Problem]

Methods of separating a target component using magnetic particles are widely used with gene detection methods and the like, for example. There is a certain need for such gene detection methods in the medical field, the agricultural field, and the food and beverage field. In a small-scale hospital, farm, or restaurant, having adequate research facilities and specialized inspectors is unrealistic in many cases, and therefore an inspection method that can be performed easily and rapidly is demanded.

In light of such an issue, the problem to be solved by the present invention is to provide a means of improving the ease and rapidity of testing using magnetic particles.

Also, in methods of the related art that use magnetic particles, the magnetic particles are made to aggregate on a wall or floor of a container by an external magnetic field, the supernatant of the liquid sample is removed, and after washing as necessary, the external magnetic field is released, causing the magnetic particles to be resuspended in the liquid or the like used for detection. In other words, because the step of removing the supernatant involves movement of the liquid sample, in cases where the liquid sample is a contaminating or infectious substance, there is a risk of contamination or infection of the inspector due to splashing of the liquid sample, aerosol diffusion, or the like.

In light of such an issue, an object of a preferred aspect of the present invention is to provide technology that reduces the risk of contamination and infection for a test using magnetic particles.

### [Solution to Problem]

The present invention that solves the above problem is a method comprising:
an affixing step of suspending magnetic particles in a liquid sample, and affixing a target component to the magnetic particles;
a recovery step of causing a magnetic collecting means to contact the liquid sample, causing the magnetic particles with the affixed target component to adhere to the magnetic collecting means by magnetism, and then recovering the magnetic collecting means from the liquid sample; and
an action step of causing the magnetic collecting means with the adhering magnetic particles to contact a liquid that acts on the target component, and causing the liquid to act on the target component in a state in which the magnetic particles are adhering to the magnetic collecting means.

The method of the present invention excels in ease and rapidity because the magnetic particles are not resuspended in the liquid, and the liquid is made to act on the target component in a state in which the magnetic particles are adhered to the magnetic collecting means.

In a preferred aspect of the present invention, the magnetic collecting means is provided on an underside of a cover,
the liquid is contained in a liquid-containing vessel having an opening that is sealable by the cover, and the action step is performed by sealing the opening of the liquid-containing vessel with the cover, causing the magnetic collecting means provided on the underside of the cover to contact the liquid.

By taking such an aspect, the step of causing the magnetic particles to contact the liquid to cause the liquid to act on the target component and the step of constructing a configuration that prevents the liquid or the target component dissolved in the liquid from splashing out of the liquid-containing vessel can be performed at the same time with the one-step procedure of sealing the liquid-containing vessel with the cover.

In cases where the liquid needs to be heated, it is necessary to take a configuration that prevents the liquid from evaporating during heating and diffusing to the outside of the liquid-containing vessel. Consequently, the present invention with such an aspect is particularly effective in cases where the liquid needs to be heated in order to act on the target component.

The step of causing the magnetic particles to contact the liquid to cause the liquid to act on the target component and the step of constructing a configuration that prevents the liquid or the target component dissolved in the liquid from splashing out of the liquid-containing vessel or diffusing by evaporation can be performed at the same time with the one-step procedure of sealing the liquid-containing vessel with the cover.

In a preferred aspect of the present invention, the magnetic collecting means is a magnetic bar having magnetic properties on at least a lower end of the magnetic bar.

By adopting a magnetic bar as the magnetic collecting means, the recovery step and the action step can be performed easily.

In a preferred aspect of the present invention, the liquid is divided into at least two or more reagents contained in the liquid-containing vessel having an opening, and at least one reagent is isolated from the other reagent or reagents by a rupturable membrane inside the liquid-containing vessel, and the action step is performed by inserting the magnetic bar with adhering the magnetic particles into the liquid-containing vessel through the opening and rupturing the membrane with the magnetic bar, causing the at least two or more reagents contained in the liquid-containing vessel to be mixed to prepare the liquid, and also causing the magnetic particles adhering to the magnetic bar to contact the liquid.

By taking such a configuration, the step of preparing the liquid by mixing reagents and the step of causing the magnetic particles to contact the liquid to cause the liquid to act on the target component can be performed at the same time with the one-step procedure of rupturing the membrane with the magnetic bar.

In a preferred aspect of the present invention, at least one membrane that vertically partitions an internal space of the liquid-containing vessel is provided inside the liquid-containing vessel,
at least one of the reagents is stored in a bottom of the liquid-containing vessel, and at least one other of the reagents is stored above the membrane, and the action step is performed by rupturing the membrane with the magnetic bar, causing the reagent or reagents stored above the membrane to run down to the bottom of the liquid-containing vessel and mix with the reagent or reagents stored in the bottom of the liquid-containing vessel to prepare the liquid, and also causing the magnetic particles adhering to the magnetic bar to contact the liquid.

According to such a configuration, the preparation of the liquid by rupturing the membrane and mixing the reagents can be performed more easily.

In addition, the present invention also relates to a testing set for recovering a target component from a liquid sample using magnetic particles, and causing a liquid to act on the target component.

That is, the present invention is a testing set comprising: a liquid-containing vessel for containing the liquid; and
a magnetic collecting means that is insertable into the liquid-containing vessel and that collects the magnetic particles by magnetism (being limited to a magnetic collecting means having no magnetism-canceling means).

According to the testing set of the present invention, because the magnetic particles are not resuspended in the liquid, and the liquid is made to act on the target component in a state in which the magnetic particles are adhered to the magnetic collecting means, easy and rapid testing can be achieved.

In a preferred aspect of the present invention, the liquid is contained in the liquid-containing vessel in advance.

By taking such a configuration, the labor of preparing the liquid is unnecessary, and a further improvement in ease and rapidity can be achieved.

In a preferred aspect of the present invention, the magnetic collecting means is a magnetic bar provided on an underside of a cover and having magnetic properties on at least a lower end,
an opening of the liquid-containing vessel is sealable by the cover, and
the magnetic bar is containable inside the liquid-containing vessel when the opening of the liquid-containing vessel is sealed by the cover.

By taking such an aspect, the step of causing the magnetic particles to contact the liquid to cause the liquid to act on the target component and the step of constructing a configuration that prevents the liquid or the target component dissolved in the liquid from splashing out of the liquid-containing vessel can be performed at the same time with the one-step procedure of sealing the liquid-containing vessel with the cover.

In a preferred aspect of the present invention, in a state before use, the magnetic collecting means is packaged by a packaging means, and the opening of the liquid-containing vessel is sealed by a temporary lid.

By packaging the magnetic collecting means in the state before use in this way, contamination can be prevented.

In cases where the liquid needs to be heated, it is necessary to take a configuration that prevents the liquid from diffusing by evaporation during heating. Consequently, the testing set of the present invention provided with a configuration provided with a magnetic bar on the underside of the cover is particularly effective in cases where the liquid needs to be heated in order to act on the target component.

The step of causing the magnetic particles to contact the liquid to cause the liquid to act on the target component and the step of constructing a configuration that prevents the liquid or the target component dissolved in the liquid from splashing out of the liquid-containing vessel or diffusing by evaporation can be performed at the same time with the one-step procedure of sealing the liquid-containing vessel with the cover.

In a preferred aspect of the present invention, at least one membrane that vertically partitions an internal space of the liquid-containing vessel and that is rupturable by the magnetic bar is provided inside the liquid-containing vessel,
the liquid is divided into at least two or more reagents contained in the liquid-containing vessel, with at least one of the reagents being stored in a bottom of the liquid-containing vessel, and at least one other of the reagents being stored above the membrane, and by rupturing the membrane, the reagent or reagents stored above the membrane run down to the bottom of the liquid-containing vessel and mix with the reagent or reagents stored in the bottom of the liquid-containing vessel, thereby preparing the liquid.

By taking such an aspect, the step of rupturing the membrane to mix the reagents and prepare the liquid, the step of causing the magnetic particles to contact the liquid to cause the liquid to act on the target component and the step of constructing a configuration that prevents the liquid or the target component dissolved in the liquid from splashing out of the liquid-containing vessel can be performed at the same time with the one-step procedure of sealing the liquid-containing vessel with the cover.

### [Advantageous Effects of Invention]

According to the present invention, the ease and rapidity of testing using magnetic particles can be improved.

Also, according to a preferred aspect of the present invention, testing technology with low risk of contamination can be provided.

### [Brief Description of Drawings]

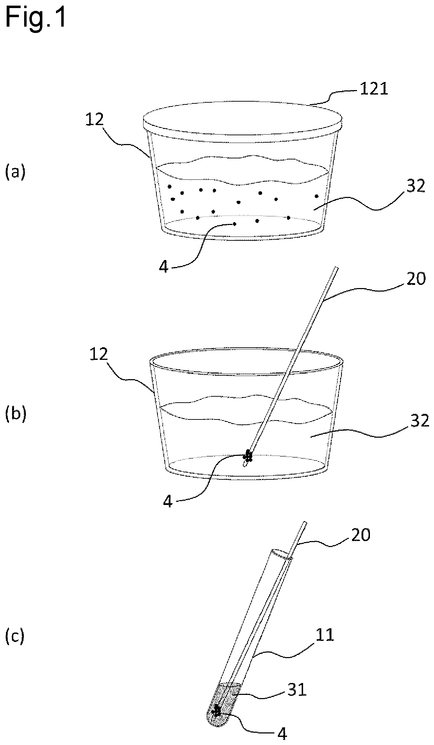

[Figure 1] FIG. 1 is a schematic diagram for explaining Embodiment 1 of the method of the present invention, in which (a) is a diagram illustrating the state of an affixing step of suspending magnetic particles 4 in a liquid sample 32 inside a liquid sample vessel 12, and affixing a target component to the magnetic particles 4, (b) is a diagram illustrating a state in which a magnetic bar 20 is made to contact the liquid sample 32 and cause the magnetic particles 4 to adhere to the magnetic bar 20 by magnetism, and (c) is a diagram explaining a state in which the magnetic bar 20 with adhering magnetic particles 4 is immersed in a liquid 31, causing the liquid 31 to act on the target component affixed to the magnetic particles 4.

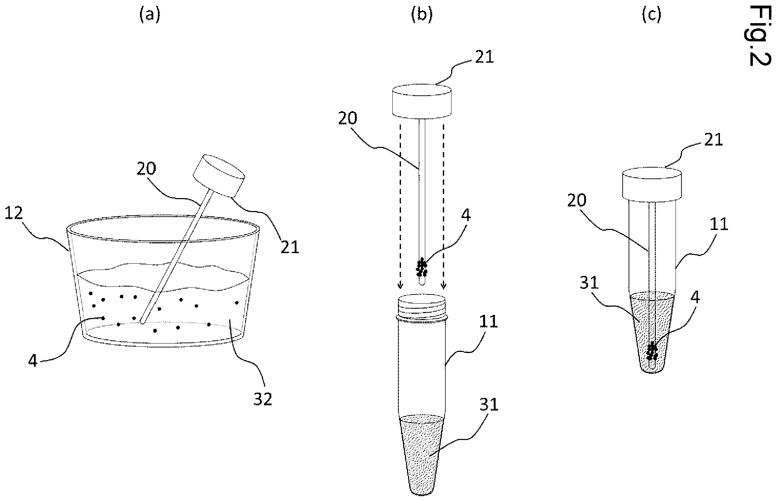

[Figure 2] FIG. 2 is a schematic diagram for explaining Embodiment 2 of the method of the present invention, in which (a) is a diagram illustrating a state in which the magnetic bar 20 provided with a cover 21 is made to contact the liquid sample 32 in which the magnetic particles 4 are suspended, and recover the magnetic particles 4, (b) is a diagram illustrating a state in which the magnetic bar 20 with adhering magnetic particles 4 is inserted into a liquid-containing vessel 11 containing the liquid 31, and (c) is a diagram illustrating a state in which an opening of the liquid-containing vessel 11 is covered by the cover 21, causing the magnetic particles 4 adhering to the magnetic bar 20 to contact the liquid 31 and causing the liquid 31 to act on the target component.

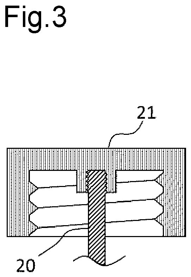

[Figure 3] FIG. 3 is a cross-section view illustrating the magnetic bar 20 provided with the cover 21 in Embodiment 2.

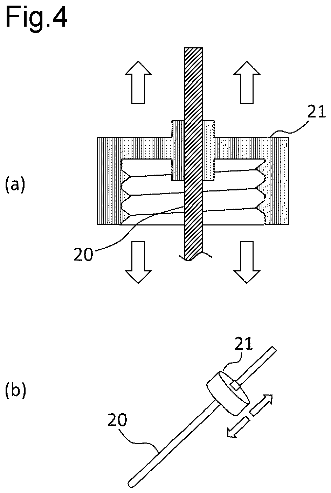

[Figure 4] FIG. 4 is a diagram illustrating the magnetic bar 20 provided with the cover 21 in a different embodiment, in which (a) is a cross-section view, and (b) is a perspective view. The white arrows indicate the slidable directions of the cover 21.

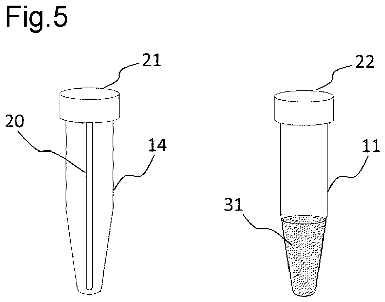

[Figure 5] FIG. 5 is a diagram illustrating a testing set of Embodiment 2 in the state before use.

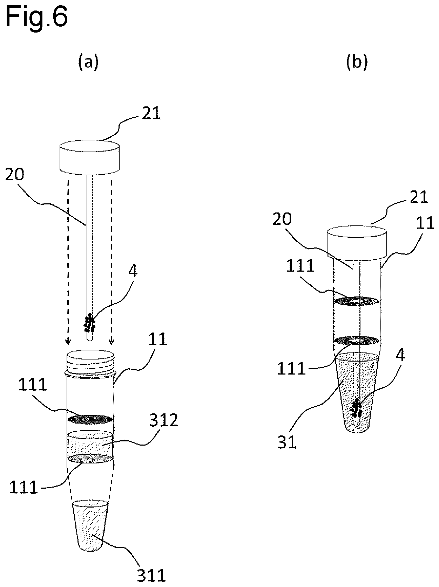

[Figure 6] FIG. 6 is a diagram illustrating the action step in Embodiment 3, in which (a) is a diagram illustrating a state in which the magnetic bar 20 with adhering magnetic particles 4 is inserted into the liquid-containing vessel 11 in which a reagent 311 and a reagent 312 are stored in isolation by membranes 111, and (b) is a diagram illustrating a state in which two membranes 111 are ruptured by the magnetic bar 20, the opening of the liquid-containing vessel 11 is sealed by the cover 21, the magnetic particles 4 adhering to the magnetic bar 20 are made to contact the liquid 31 obtained by the mixture of the reagent 311 and the reagent 312, and the liquid 31 is made to act on the target component.

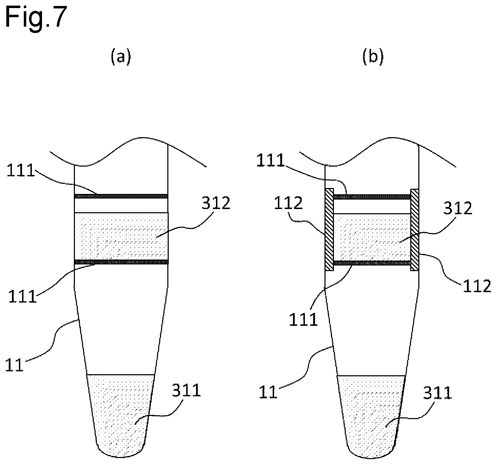

[Figure 7] FIG. 7 illustrates a cross-section view of the liquid-containing vessel 11 in which the reagent 311 and the reagent 312 are stored in isolation by the membranes 111, in which (a) illustrates a cross-section view of the liquid-containing vessel 11 of Embodiment 3, and (b) illustrates a cross-section view of the liquid-containing vessel 11 in a different embodiment.

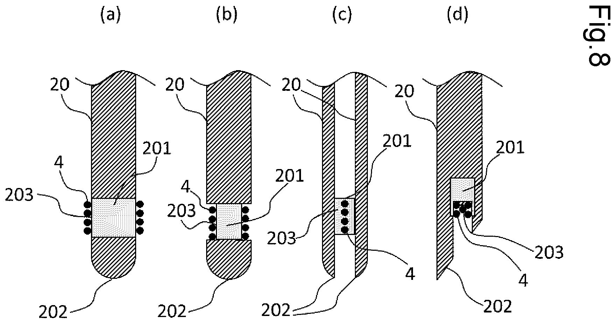

[Figure 8] FIG. 8 is a cross-section view of the tip portion of the magnetic bar 20.

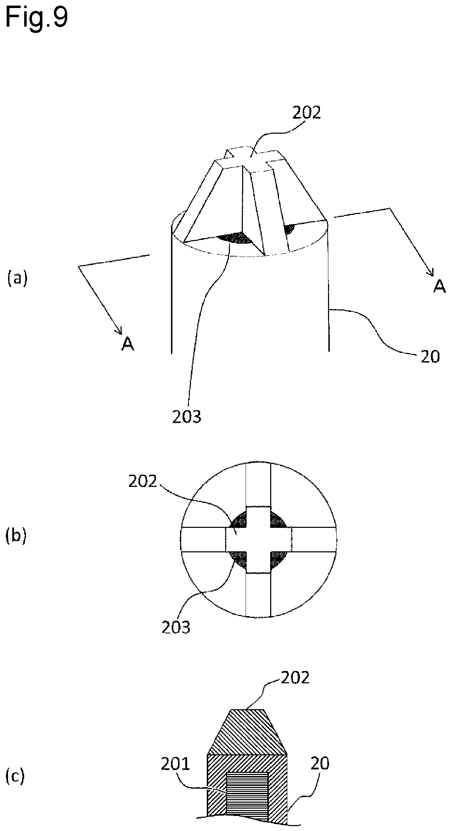

[Figure 9] FIG. 9 is a diagram illustrating the tip portion of the magnetic bar 20, in which (a) illustrates a perspective view of the tip portion, (b) illustrates a plan view, and (c) illustrates a cross-section view along the line AA.

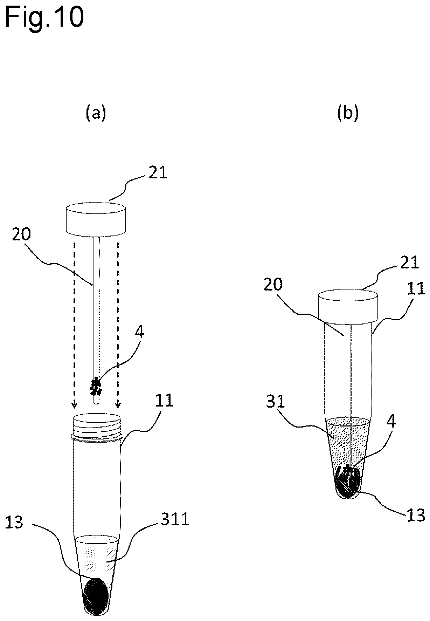

[Figure 10] FIG. 10 is a diagram illustrating the action step in Embodiment 4, in which (a) is a diagram illustrating a state in which the magnetic bar 20 with adhering magnetic particles 4 is inserted into the liquid-containing vessel 11 containing the reagent 311 and a bag 13 formed by the membranes 111 storing the reagent 312, and (b) is a diagram illustrating a state in which the bag 13 is ruptured by the magnetic bar 20, the opening of the liquid-containing vessel 11 is sealed by the cover 21, the magnetic particles 4 adhering to the magnetic bar 20 are made to contact the liquid 31 obtained by the mixture of the reagent 311 and the reagent 312, and the liquid 31 is made to act on the target component.

### [Description of Embodiments]

### <Embodiment 1>

Hereinafter, Embodiment 1 of the method of the present invention will be described with reference to FIG. 1.

In Embodiment 1, first, magnetic particles 4 are suspended in a liquid sample 32 (FIG. 1(a).

The specific form of the liquid sample 32 to which the present invention is applicable is not particularly limited, and is sufficiently a liquid containing a target component that is targeted for isolation or detection. Examples of the liquid sample include biologically-derived liquid samples such as blood, sweat, and urine, environmentally-derived liquid samples such as river water, seawater, and suspensions of soil, and liquid samples derived from food products.

The type of the magnetic particles 4 is not particularly limited insofar as the magnetic particles 4 can be suspended in a liquid and possible by a magnetic collecting means. For example, commercial products such as DYNABEADS (registered trademark) (Invitrogen), PureProteome (Merck Millipore), magnetic beads obtainable from New England Biolabs, and Mag Sepharose (GE Healthcare) can be used.

The target component likewise is not particularly limited, and can be set according to the purpose of testing. Specific examples include cellular material such as animal cells, plant cells, and bacteria, biomolecules such as nucleic acids, proteins, saccharides, and lipids, natural small molecules, synthetic small molecules, high molecular compounds, and metals.

The magnetic particles 4 need to be particles to which the target component can be affixed. A configuration enabling the target component to be affixed can be set appropriately depending on the type of the target component.

For example, magnetic particles 4 coated with a substance having a direct or indirect binding ability with respect to the target component can be used, such as antibodies or antibody fragments, protein A, protein G, protein L, peptides having a tag sequence, streptavidin, biotin, nucleic acid binding proteins, nucleic acids, nucleotides, aptamers, PNA, enzymes, or other organic compounds.

The method of coating the magnetic particles 4 may follow conventional methods. Additionally, commercially available pre-coated magnetic particles may also be used.

The method of suspending the magnetic particles 4 in the liquid sample 32 is not particularly limited. As illustrated in FIG. (a), a liquid mixture may be contained inside a liquid sample vessel 12 sealed by a lid 121 and shaken, or stirred with a stirrer. The form of the liquid sample vessel 12 is not limited to the illustration in FIG. 1(a), and any of various types of vessels may be selected and used appropriately.

In the method of Embodiment 1, as illustrated in FIG. 1(b), the magnetic bar 20 is made to contact the liquid sample 32 in which the magnetic particles 4 are suspended, causing the magnetic particles 4 to adhere to the magnetic bar 20 by magnetism.

The configuration of the magnetic bar 20 is not particularly limited, and is sufficiently a configuration allowing recovered magnetic particles 4 to contact a liquid 31 in the action step described later.

As illustrated in FIG. 1(b), it is preferable to use a magnetic bar 20 provided with a magnetic part that is magnetized near the lower end.

The magnetic part can be formed using a permanent magnet such as an alnico magnet, a ferrite magnet, a neodymium magnet, or a samarium cobalt magnet.

In the step of recovering the magnetic particles 4, in order to recover the magnetic particles 4 efficiently, it is preferable to stir the liquid sample 32 with the magnetic bar 20.

After the magnetic particles 4 are made to adhere to the magnetic bar 20, the magnetic bar 20 is lifted out of the liquid sample 32 and the magnetic particles 4 are recovered from the liquid sample 32.

After recovery, a cleaning step of immersing the tip of the magnetic bar 20 in a cleaning solution and cleaning the magnetic particles 4 may also be included as necessary.

After the recovery step, the magnetic bar 20 is immersed in the liquid 31 contained in a liquid-containing vessel 11, causing the magnetic particles 4 to contact the liquid 31 and causing the liquid 31 to act on the target component (FIG. 1(c)).

Herein, "action" includes not only chemical reactions that enable detection of the target component, but also cellular growth action as typified by the action of a culture medium on cells.

The type of the liquid 31 and the form of the action of the liquid 31 on the target component may be selected appropriately according to the type of the target component and the purpose of the testing.

For example, in the case where the purpose of the testing is to detect the target component, the liquid 31 is taken to be a detection liquid that exhibits a detection reaction with respect to the target component.

Also, in the case where the purpose of the testing is to isolate and cultivate cells or bacteria for example, the liquid 31 is taken to be a culture medium used for cultivation.

In FIG. 1(c), a test tube is illustrated as the liquid-containing vessel 11, but the form thereof is not particularly limited, and may be selected appropriately according to the purpose of the testing.

In the case where the liquid-containing vessel 11 has an opening that is open like the test tube illustrated in FIG. 1(c), the opening may be covered with a thin film such as aluminum foil or plastic wrap as necessary to perform the action step.

### <Embodiment 2>

Hereinafter, Embodiment 2 of the present invention will be described with reference to FIGS. 2 to 5. Note that matters which are also suited to Embodiment 2 from among the matters described in the description of Embodiment 1 can also be applied to Embodiment 2.

In Embodiment 2, a magnetic bar 20 integrated with a cover 21 is used as the magnetic collecting means. The magnetic bar 20 is provided on the underside of the cover 21. The cover 21 is configured to be capable of sealing the opening of the liquid-containing vessel 11.

As illustrated in FIG. 3, the magnetic bar 20 in Embodiment 2 is fitted onto the underside of the cover 21, but the magnetic bar 20 and the cover 21 may also be configured as a single member for example.

The specific configuration of providing the magnetic bar 20 on the underside of the cover 21 is not particularly limited, but as illustrated in FIG. 4(a) for example, the magnetic bar 20 may be provided by penetrating and compressively fitting in a through-hole passing through both sides of the cover 21. By taking such a configuration, the cover 21 can be configured to be capable of sliding in the axial direction of the magnetic bar 20, as illustrated in FIGS. 4(a) and 4(b).

According to this configuration, for example, a method can be achieved in which, in the recovery step, the cover 21 is positioned near the upper end of the magnetic bar 20, and in the action step, the cover 21 is made to slide toward the lower end of the magnetic bar 20 and seal the opening of the liquid-containing vessel 11.

In the recovery step, the tester is able to operate the magnetic bar 20 while gripping the cover 21. Because the recovery step can be performed without directly touching the magnetic bar 20, the risk of contamination can be lowered.

After the completion of the recovery step, the magnetic bar 20 with adhering magnetic particles 4 is inserted from the opening of the liquid-containing vessel 11 (FIG. 2(b)), and the opening of the liquid-containing vessel 11 is sealed by the cover 21 (FIG. 2(c)).

In Embodiment 2, the cover 21 is a screw cap, and is capable of sealing the opening of the liquid-containing vessel 11 by being screwed onto the opening (FIG. 3). The cover 21 is not required to be a screw cap, and the form thereof is not particularly limited insofar as the opening of the liquid-containing vessel 11 can be sealed.

In association with the movement of inserting the magnetic bar 20 and sealing the opening of the liquid-containing vessel 11 with the cover 21, the magnetic particles 4 adhering by magnetism near the lower end of the magnetic bar 20 come into contact with the liquid 31 contained in the liquid-containing vessel 11. Also, at the same time, in the action step, a sealed configuration that prevents the liquid 31 from splashing outside from the opening of the liquid-containing vessel 11 is completed.

In other words, in Embodiment 2, contact with the liquid 31 by the magnetic particles 4 and the construction of a structure preventing splashing of the liquid 31 can be achieved with the one-step procedure of sealing the opening of the liquid-containing vessel 11 with the cover 21. According to Embodiment 2, testing that is easy and rapid and furthermore has a low risk of contamination can be achieved.

In the case where heating is necessary for the liquid 31 to act on the target component, there is a risk that the heated liquid 31 will diffuse by evaporation, and diffuse to the outside from the liquid-containing vessel 11. In Embodiment 2 of the present invention, because the opening of the liquid-containing vessel 11 can be sealed by the cover 21, evaporative diffusion of the liquid 31 due to heating can be prevented.

In other words, in the case where heating is necessary for the liquid 31 to act on the target component, contact with the liquid 31 by the magnetic particles 4 and a structure preventing splashing and evaporative diffusion of the liquid 31 can be achieved with the one-step procedure of sealing the opening of the liquid-containing vessel 11 with the cover 21.

When the temperature of the liquid 31 when acting on the target component is 30°C or higher, preferably 40°C or higher, more preferably 50°C or higher, it is preferable to apply Embodiment 2 of the present invention from the perspective of preventing evaporative diffusion of the liquid 31.

The magnetic bar 20 provided with the cover 21 and the liquid-containing vessel 11 are preferably provided as a combined testing set.

The liquid 31 contained in the liquid-containing vessel 11 may also take the form of a liquid prepared by the tester at the time of use. From the perspective of achieving easy and rapid testing, it is preferable to provide the liquid 31 already contained inside the liquid-containing vessel 11 (FIG. 5). In this case, the opening of the liquid-containing vessel 11 is preferably provided in a state of being sealed by a temporary lid 22 (FIG. 5).

Also, because it is not preferable for the magnetic bar 20 to recklessly contact the external environment before the testing set is used, the magnetic bar 20 is preferably packaged by some kind of packaging means. The packaging means may be a protective vessel having an opening that is sealable by the cover 21 (FIG. 5). As illustrated in FIG. 5, before use, the magnetic bar 20 is preferably provided in a state of being contained in a protective vessel 14 and with the cover 21 sealing the opening of the protective vessel 14.

The packaging means for the magnetic bar 20 before use is not limited to the form illustrated in FIG. 5, and may also be an individual packaging or a grouped packaging. In the case of the form illustrated in FIG. 5, the cover 21 is exposed to the external environment, but packaging that protects not only the magnetic bar 20 but also the cover 21 may also be used.

For the testing set, it is sufficient to include the magnetic bar 20 provided with the cover 21 and the liquid-containing vessel 11, but otherwise, a form is possible in which the magnetic particles 4, a vessel containing the magnetic particles 4 and a liquid sample vessel for containing the liquid sample are also provided together as elements of the testing set.

### <Embodiment 3>

Hereinafter, Embodiment 3 of the present invention will be described with reference to FIGS. 6 to 9. Note that matters which are also suited to Embodiment 3 from among the matters described in the description of Embodiments 1 and 2 can also be applied to Embodiment 3.

In Embodiment 3, two membranes 111 that vertically partition the internal space of the liquid-containing vessel 11 are provided inside the liquid-containing vessel 11 (FIGS. 6 and 7). Additionally, a reagent 312 is stored in the space demarcated by the membranes 111 and the walls of the liquid-containing vessel 11, while a reagent 311 is stored in the bottom of the liquid-containing vessel 11 (FIGS. 6 and 7).

The liquid 31 can be prepared by mixing the reagent 311 and the reagent 312. Since it is sufficient for the mixed liquid 31 to be a liquid, either of the reagent 311 and the reagent 312 may also be solid.

FIGS. 6 and 7 disclose a configuration provided with two membranes 111, but a configuration provided with a single membrane 111 is also possible. Furthermore, a configuration is possible in which three or more membranes 111 are provided and the liquid 31 is divided into three or more reagents that are contained in the liquid-containing vessel 11.

The material of the membranes 111 is not particularly limited insofar as the membrane is rupturable by the magnetic bar 20, and may be aluminum foil, paper, or resin.

The membranes 111 may be provided directly on the walls of the liquid-containing vessel 11 (FIG. 7(a)) or provided by fitting a disc member 112 across which the membranes 111 are stretched inside the liquid-containing vessel 11 (FIG. 7(b)).

Because the membranes 111 are rupturable by the magnetic bar 20, if the magnetic bar 20 with adhering magnetic particles 4 is inserted into the liquid-containing vessel 11 (FIG. 6(a)) and the opening of the liquid-containing vessel 11 is sealed by the cover 21, the membranes 111 are ruptured, and the reagent 312 stored above runs down to the bottom of the liquid-containing vessel 11 (FIG. 6(b)). Then, the reagent 312 mixes with the reagent 311 stored in the bottom of the liquid-containing vessel 11, causing the liquid 31 to be prepared, while simultaneously, the magnetic particles 4 adhering to the magnetic bar 20 contact the prepared liquid 31, and the action of the liquid 31 on the target component can be started.

In other words, in Embodiment 3, the preparation of the liquid 31, contact with the liquid 31 by the magnetic particles 4, and the construction of a structure preventing splashing of the liquid 31 can be achieved with the one-step procedure of sealing the opening of the liquid-containing vessel 11 with the cover 21.

The present invention according to Embodiment 3 is highly effective in cases where the liquid 31 is a liquid that needs to be prepared at the time of use, particularly when the liquid 31 is a reaction liquid used in a known detection reaction for which a non-specific reaction occurs unless the liquid is prepared at the time of use.

In Embodiment 3, it is necessary to rupture the membranes 111 using the magnetic bar 20 with adhering magnetic particles 4. The rupturing of the membranes 111 by the magnetic bar 20 is achieved specifically by pressing a rupture site 202 such as the tip of the magnetic bar 20 against the membranes 111, but if the magnetic particles 4 are adhering to the rupture site 202, there is a chance that the magnetic particles 4 will be detached due to contact with the membranes 111.

To prevent the magnetic particles 4 from detaching, as illustrated in FIG. 8(a), a magnetic particle adherence site 203 where the magnetic particles 4 adhere is preferably distanced from the rupture site 202. The position of the magnetic particle adherence site 203 can be suitably designed according to how a magnetic part 201 is installed in the magnetic bar 20. In Embodiment 3, the magnetic particle adherence site 203 is configured to be distanced from the rupture site 202 by providing the magnetic part 201 having magnetic properties at a predetermined distance from the tip of the magnetic bar 20.

Also, as illustrated in FIG. 8(b), the diameter of the magnetic part 201 is preferably narrower than the portions other than the magnetic part 201 of the magnetic bar 20, or the magnetic bar 20 is preferably provided inside a depression provided in the magnetic bar 20. By taking such a configuration, it is possible to prevent the magnetic particles 4 from detaching due to contact with the ruptured membranes 111.

As illustrated in FIG. 8(c), the magnetic bar 20 may contain two bar members configured with the magnetic part 201 interposed in between.

The tips of the magnetic bar 20 may also be given a sharp slope, and the magnetic part 201 may be recessed inside a depressed part provided in the slope. By taking such a configuration, it is easy to rupture the membranes 111 with the magnetic bar 20, and in addition, the magnetic particles 4 can be prevented from detaching from the magnetic bar 20 due to contact with the membranes 111 (FIG. 8(d)).

FIGS. 8(a) to 8(d) illustrate an example of a configuration in which the magnetic part 201 having magnetic properties is provided a predetermined distance from the tip of the magnetic bar 20. However, if the magnetic particle adherence site 203 can be configured to be distanced from the rupture site 202, it is not strictly necessary to provide the magnetic part 201 at a predetermined distance from the tip of the magnetic bar 20.

For example, even in the case where the tip of the magnetic bar 20 is configured as the magnetic part 201, if the magnetic part 201 itself is provided with a depression and the magnetic particles 4 are accumulated inside the depression, a configuration in which the magnetic particle adherence site 203 is distanced from the rupture site 202 can be achieved.

Otherwise, the configuration for separating the rupture site 202 and the magnetic particle adherence site 203 from each other may also be a configuration in which the magnetic part 201 is buried inside the magnetic bar 20, and a protruding member equipped with the rupture site 202 is provided on the tip (FIG. 9).

As illustrated in FIG. 9(a), the protruding member takes a concave polygonal frustum shape extending in the tip direction from a base part of the magnetic bar 20. As illustrated in FIG. 9(c), because of magnetism of the magnetic part 201 buried inside the magnetic bar 20, the root portion of the protruding member acts as the magnetic particle adherence site 203 (FIGS. 9(a) and 9(b)).

Although FIG. 9 illustrates a concave polygonal frustum shape as the protruding member, it is sufficient for the protruding member to have a margin allowing the magnetic particle adherence site 203 to be formed, and the protruding member may also have a concave polygonal pyramid shape.

### <Embodiment 4>

Hereinafter, Embodiment 4 of the present invention will be described with reference to FIG. 10. Note that matters which are also suited to Embodiment 4 from among the matters described in the description of Embodiments 1 and 3 can also be applied to Embodiment 4.

In Embodiment 4, the reagent 311 and a bag 13 formed using the membrane 111 are contained in the liquid-containing vessel 11. Furthermore, the reagent 312 is contained inside the bag 13.

Because the membrane 111 is rupturable by the magnetic bar 20, if the magnetic bar 20 with adhering magnetic particles 4 is inserted into the liquid-containing vessel 11 (FIG. 10(a)) and the opening of the liquid-containing vessel 11 is sealed by the cover 21, the bag 13 is ruptured, and the reagent 312 contained inside leaks out (FIG. 10(b)). Then, the reagent 312 mixes with the reagent 311 stored in the bottom of the liquid-containing vessel 11, causing the liquid 31 to be prepared, while simultaneously, the magnetic particles 4 adhering to the magnetic bar 20 contact the prepared liquid 31, and the action of the liquid 31 on the target component can be started.

In other words, in Embodiment 4, like Embodiment 3, the preparation of the liquid 31, contact with the liquid 31 by the magnetic particles 4, and the construction of a structure preventing splashing of the liquid 31 can be achieved with the one-step procedure of sealing the opening of the liquid-containing vessel 11 with the cover 21.

The present invention according to Embodiment 4 is highly effective in cases where the liquid 31 is a liquid that needs to be prepared at the time of use, particularly when the liquid 31 is a reaction liquid used in a known detection reaction for which a non-specific reaction occurs unless the liquid 31 is prepared at the time of use.

### [Industrial Applicability]

The present invention can be applied to inspection equipment.

### [Reference Signs List]

- 11: liquid-containing vessel
- 111: membrane
- 12: liquid sample vessel
- 121: lid
- 13: bag
- 14: protective vessel
- 20: magnetic bar
- 201: magnetic part
- 202: rupture site
- 203: magnetic particle adherence site
- 21: cover
- 31: liquid
- 311: reagent
- 312: reagent
- 32: liquid sample
- 4: magnetic particle

## Claims

1. A method comprising:
an affixing step of suspending magnetic particles in a liquid sample, and affixing a target component to the magnetic particles;
a recovery step of causing a magnetic collecting means to contact the liquid sample, causing the magnetic particles with the affixed target component to adhere to the magnetic collecting means by magnetism, and then recovering the magnetic collecting means from the liquid sample; and
an action step of causing the magnetic collecting means with the adhering magnetic particles to contact a liquid that acts on the target component, and causing the liquid to act on the target component in a state in which the magnetic particles are adhering to the magnetic collecting means.

2. The method of claim 1, wherein
the magnetic collecting means is provided on an underside of a cover,
the liquid is contained in a liquid-containing vessel having an opening that is sealable by the cover, and
the action step is performed by sealing the opening of the liquid-containing vessel with the cover, causing the magnetic collecting means provided on the underside of the cover to contact the liquid.

3. The method of claim 2, wherein
the liquid needs to be heated to act on the target component.

4. The method according to any one of claims 1 to 3, wherein
the magnetic collecting means is a magnetic bar having magnetic properties on at least a lower end of the magnetic bar.

5. The method of claim 4, wherein
the liquid is divided into at least two or more reagents contained in the liquid-containing vessel having an opening, and at least one reagent is isolated from the other reagent or reagents by a rupturable membrane inside the liquid-containing vessel, and
the action step is performed by inserting the magnetic bar with adhering the magnetic particles into the liquid-containing vessel through the opening and rupturing the membrane with the magnetic bar, causing the at least two or more reagents contained in the liquid-containing vessel to be mixed to prepare the liquid, and also causing the magnetic particles adhering to the magnetic bar to contact the liquid.

6. The method of claim 5, wherein
at least one membrane that vertically partitions an internal space of the liquid-containing vessel is provided inside the liquid-containing vessel,
at least one of the reagents is stored in a bottom of the liquid-containing vessel, and at least one other of the reagents is stored above the membrane, and
the action step is performed by rupturing the membrane with the magnetic bar, causing the reagent or reagents stored above the membrane to run down to the bottom of the liquid-containing vessel and mix with the reagent or reagents stored in the bottom of the liquid-containing vessel to prepare the liquid, and also causing the magnetic particles adhering to the magnetic bar to contact the liquid.

7. A testing set for recovering a target component from a liquid sample using magnetic particles, and causing a liquid to act on the target component, the testing set comprising:
a liquid-containing vessel for containing the liquid; and
a magnetic collecting means that is insertable into the liquid-containing vessel and that collects the magnetic particles by magnetism (being limited to a magnetic collecting means having no magnetism-canceling means).

8. The testing set according to claim 7, wherein
the liquid is contained in the liquid-containing vessel in advance.

9. The testing set according to claim 8, wherein
the magnetic collecting means is a magnetic bar provided on an underside of a cover and having magnetic properties on at least a lower end,
an opening of the liquid-containing vessel is sealable by the cover, and
the magnetic bar is containable inside the liquid-containing vessel when the opening of the liquid-containing vessel is sealed by the cover.

10. The testing set according to claim 9, wherein
in a state before use, the magnetic collecting means is packaged by a packaging means, and the opening of the liquid-containing vessel is sealed by a temporary lid.

11. The testing set according to claim 9 or 10, wherein
the liquid needs to be heated to act on the target component.

12. The testing set according to any one of claims 9 to 11, wherein
at least one membrane that vertically partitions an internal space of the liquid-containing vessel and that is rupturable by the magnetic bar is provided inside the liquid-containing vessel,
the liquid is divided into at least two or more reagents contained in the liquid-containing vessel, with at least one of the reagents being stored in a bottom of the liquid-containing vessel, and at least one other of the reagents being stored above the membrane, and
by rupturing the membrane, the reagent or reagents stored above the membrane run down to the bottom of the liquid-containing vessel and mix with the reagent or reagents stored in the bottom of the liquid-containing vessel, thereby preparing the liquid.
